# EUROPEAN PATENT SPECIFICATION

(11) **EP 1 206 928 B1**
(45) Date of publication and mention of the grant of the patent: **28.09.2011**
(21) Application number: 00953468.6
(22) Date of filing: 17.08.2000
(51) Int. Cl.: A61Q 15/00, C12G 1/00, C12G 3/00, C08J 3/12, A23L 1/0532, A23L 1/0522, A23L 1/0562

(54) **WATER-CONTAINING POWDER COMPOSITION, PROCESS FOR PRODUCING THE SAME, AND COSMETIC PREPARATION CONTAINING THE POWDER COMPOSITION**
WASSERENTHALTENDE PUDERZUBEREITUNG, VERFAHREN ZU DEREN HERSTELLUNG UND KOSMETISCHE ZUBEREITUNG DIESE ENTHALTEND
COMPOSITION DE POUDRE A BASE D'EAU, PROCEDE DE FABRICATION ET PREPARATION COSMETIQUE RENFERMANT LADITE COMPOSITION

(30) Priority: 24.08.1999 JP 23714999; 08.06.2000 JP 2000172172
(43) Date of publication of application: 22.05.2002
(73) Proprietor: Kosé Corporation, Chuo-ku, Tokyo 103-0027 (JP)
(72) Inventor: TANAKA, Yoichiro, Tokyo 114-0005 (JP); TOMITA, Yuriko, Tokyo 114-0005 (JP); KOBAYASHI, Shinji, Tokyo 114-0005 (JP)
(74) Representative: Hartz, Nikolai
(86) International application number: PCT/JP2000/005510
(87) International publication number: WO 2001/013864

(56) References cited:
- FR-A- 1 334 917
- JP-A- 4 001 118
- JP-A- 54 104 487
- JP-A- 59 046 124
- JP-A- 59 046 125
- US-A- 3 242 051
- US-A- 3 405 071

## Description

### TECHNICAL FIELD

The present invention relates to a composition in which water is powderized and, more particularly, to a water-containing powder composition that is superior in production stability and storage stability, and releases water when pressed with the fingers or the like during use. The water-containing powder composition is widely utilizable in cosmetics, foods, perfumes, agricultural chemicals and medicines.

### BACKGROUND ART

The inventors of the present invention previously developed a water-containing powder composition that releases water when pressed with the fingers or the like during use, and filed a Patent application (Japanese Patent Application laid-open No. 1993-65212). This water-containing powder composition, prepared by a technology for powdering water and oil using powder treated with hydrophobic silicic acid anhydride and a fluorine compound, experienced problems relating to stable production and storage stability, due to fluctuation in the degree of hydrophobicity of the silicic acid anhydride.

Therefore, the inventors of the present invention investigated the technology of constantly producing the water-containing powder composition with the above properties by using microcapsule technology.

The document US 3 405 071 discloses a water containing powder composition comprising aqueous cores. The cores are gelled with a gellant such as agar agar prior to freezing the water phase and crushing into particles. Finally, the cores are coated with a hydrophobic film of hydrophobic grafted polymer.

The inclusion of water in hydrophobic particles using microcapsule technology has been disclosed in Japanese Patent Publication 1991-67737 (JP59-046125). In this method, water is frozen, crushed into particles, and then coated with hydrophobic particles.

However, it is difficult to obtain a useful microcapsule using the above method due to the following problems: ① it is difficult to obtain a fine particle diameter when the frozen water is shattered; ② the water (ice) particles easily flocculate and associate during the process of shattering frozen water and coating the particles; and ③ flocculation or association of the shattered water (ice) particles tends to occur when the surface of the particles is coated.

Therefore, development of a technology for manufacturing a water-containing powder composition capable of releasing water when pressed with the fingers during use by improving conventional microcapsule technology without the above manufacturing problems has been desired.

### DISCLOSURE OF THE INVENTION

As a result of extensive studies to solve the above problems, the inventors of the present invention have discovered that a water-containing powder with a fine particle diameter can be obtained without the problems of flocculation and association of particles by causing an aqueous phase ingredient to gel with a specific water-soluble gellant, forming the gel into particles each serving as a core by freeze-shattering, and then coating each core with hydrophobic particles wherein the gellant is one or more components selected from the group consisting of agar, gelatin, carageenan, gellan given, and magnesium sodium silicate. The inventors have further discovered that the obtained water-containing powder displays excellent production stability and storage stability, and maintains the characteristics of releasing water when pressed with the fingers or the like during use. These findings have led to the completion of the present invention.

Specifically, the present invention provides a water-containing powder composition comprising aqueous gel cores coated with hydrophobic particles
wherein the aqueous gel cores are obtainable by
(i) gelling an aqueous phase ingredient with a water-soluble gellant, wherein the aqueous gellant is one or more components select from the group consisting of agar, gelatin, carageenan, gellan gum, and magnesium sodium silicate and
(ii) freese-shattering the gel.

The present invention further provides a process for manufacturing a water-containing powder composition comprising causing an aqueous phase ingredient to gel with a water-soluble gellant, forming the gel into particles each serving as a core by means of freeze-shattering, and coating the cores with hydrophobic particles
wherein the gellant is one or more components selected from the group consisting of agar, gelatin, carageenan, gellan gum, and magnesium sidium silicate.

Furthermore, the present invention provides a cosmetic composition comprising any one of the above mentioned water-containing powder compositions and a method of applying the cosmetic compositions to the skin.

### BEST MODE FOR CARRYING OUT THE INVENTION

The water-containing powder composition of the present invention has a structure wherein hydrophobic particles are coated on the exterior surface of aqueous gel cores. This water-containing powder composition is manufactured by causing an aqueous phase ingredient to gel with a water-soluble gellant, forming the gel into particles, each serving as a core, by freeze-shattering and then coating each core with hydrophobic particles.

The amount of water used in the aqueous gel of the water-containing powder composition of the present invention, is preferably 30-99.7 weight% (hereinafter indicated by %) and more preferably 40-98%. The water content in this range can provide an excellent fresh feel of water.

The aqueous phase ingredient for producing this aqueous gel core is an active component and other ingredients comprising water and has hydrophilic properties. As the active component, antiseptic agents such as paraoxybenzoate and phenoxy ethanol; humectants such as 1,3-butylene glycol, dipropylene glycol, ethylene glycol, glycerol, and diglycerol; fresheners such as ethanol and menthol; surfactants; and other pharmacological agents such as vitamin C derivatives can be given. As the aqueous phase ingredient, an oil-in-water emulsion in which oil is emulsified and dispersed in water, and a suspension with particles dispersed in water or an oil-in-water emulsion can also be used.

The water soluble gellant is an agent for gelling water by dissolving or swelling in water.

These may be used individually or in combination of two or more.

As the water-soluble gellant, from the viewpoint of ease in freeze-shattering, and improvement in storage stability, that form hard gels with water such as agar, gelatin, carageenan, gellan gum, and magnesium sodium silicate are used.

In order to ensure that a sufficient amount of aqueous phase ingredient can be acquired, the content of the water-soluble gellant used in the present invention is preferably 0.1-10% of the aqueous gel, varying depending on the type of water-soluble gellant used.

When the water-soluble gellant of the present invention is a compound requiring a counter-alkali for gelling water, such as carboxy vinyl polymer, alkyl modified carboxy vinyl polymer, sodium hydroxide, potassium hydroxide, triethanolamine, and diethanolamine may be used. In this instance, depending on the type of water-soluble high polymer used, the ratio of the water-soluble gellant and the alkali is preferably 1:0.001-1:1.

In the present invention, as methods for producing a powdered aqueous gel core using an aqueous gel composed of an aqueous phase ingredient and a water soluble gellant, freeze-shattering is given. As a method of freeze shattering, freezing of the aqueous gel using a refrigerant such as liquid nitrogen and then crushing the aqueous gel can be given. For the temperature used during freeze-shattering of the aqueous gel, -20°C to -190°C is preferable depending on the hardness and coagulating point of the aqueous gel. The particle size after freeze-shattering is preferably between about 1 µm and 300 µm.

The hydrophobic particles used to coat the powdered particles of the aqueous gel obtained in this manner function to improve anti-aggregation and storage stability by adhering to or being adsorbed onto the surface. As these hydrophobic particles, particles exhibiting hydrophobicity, hydrophilic particles that are surface treated using a known hydrophobicizing agent, hydrophobic particles further treated using a hydrophobicizing agent to increase the degree of hydrophobicity can be used. The particle diameter of these hydrophobic particles must be smaller than the particle diameter of the powdered particles of the aqueous gel. A particle diameter 1/10 or less of the particle diameter of the aqueous gel powdered particles is preferable from the viewpoint of coating efficiency of the surface.

Among the hydrophobic particles used in the present invention, as examples of the particles having hydrophobicity, polystyrene powder, polyethylene powder, organopolysiloxane elastomer powder, polymethylsilsesquioxane powder, N-acyl-lysine, polyethylene tetrafluoride resin powder, acrylic resin powder, epoxy resin powder, nylon powder, aluminum stearate, zinc laurate, and magnesium stearate can be given. These particles may be used individually or in combination of two or more.

A hydrophobic powder prepared by treating the surface of a hydrophilic powder with a hydrophobicizing agent can also be used. As the hydrophobicizing agent, organic silicon compounds such as trymethylsilylization agent and methylhydrodiene polysyloxane, fluorine compounds such as perfluoropolyether alkyl phosphate and perfluoropolyether silane, metallic soaps, and oil agents can be given. These agents may be used individually or in combination of two or more. Of these, organic silicon compounds and fluorine compounds are preferable due to the improvement in the degree of hydrophobicity. As the hydrophilic particles, for example, inorganic particles such as titanium oxide, zinc oxide, silicic acid anhydride, aluminium oxide, magnesium oxide, zirconium oxide, magnesium carbonate, calcium carbonate, aluminium silicate, magnesium silicate, magnesium aluminium silicate, mica, synthetic mica, synthetic sericite, sericite, talc, silicon carbide, barium sulfate, boron nitride, bismuth oxychloride, and mica titanium; organic particles such as silk powder, starch, and cellulose crystal; and composite particles such as mica titanium coated with titanium oxide powder, zinc oxide powder, or barium sulfate can be given. These hydrophilic particles surface treated with a hydrophobicizing agent may be used individually or in combination of two or more.

Of the above-mentioned hydrophobic particles, fumed silicic acid anhydride treated by a hydrophobicizing agent with an average particle diameter of 0.001-0.1 µm is particularly preferable due to the increase in storage stability. As commercially available products of these hydrophobic particles, AEROSIL R974, R972, RX200, RX300 (manufactured by Nippon Aerosil Co., Ltd.), and CAB-O-SIL TS-530 (manufactured by Cabot Corporation) can be given. In the water-containing powder composition of the present invention, a preferable mass ratio of the aqueous gel powder and hydrophobic particles is approximately 100:0.5-100:25, although such a ratio varies according to the particle diameter of the aqueous gel powder and the hydrophobic particles.

There are no particular limitations to the method of coating the surface of the aqueous gel powder with the hydrophobic particles. One example of such a method of coating comprises placing the hydrophobic particles in a stirring vessel and adding the aqueous gel powder while stirring at a low temperature to prevent fusion or aggregation due to a rise in temperature. The stirring vessel used preferably has a jacket with a cooling mechanism and stirring blades that rarely come in contact with the wall and bottom of the jacket.

In the cosmetic preparation of the present invention, the content of the above water-containing powder composition is preferably 10-100%, and more preferably 30-90%. When this range is used, a cosmetic preparation exhibiting particularly superior freshness and a refreshing feeling, which are the effects attainable by the addition of water, can be obtained.

In addition to the above water-containing powder composition, various additives conventionally used in cosmetic preparations can be added to the cosmetic preparation containing the water-containing powder composition of the present invention to the extent the effect is not adversely affected. Such additives include powders, oil agents; surfactants; oil gelling agents such as partially cross-linked organopolysiloxane and dextrin fatty acid esters; UV absorbers; oil soluble film-forming agents such as acryl-modified silicone and trimethylsiloxysilicate; solvents such as ethanol; antiseptic agents such as para-oxybenzoic acid derivatives and phenoxyethanol; vitamins; antiphlogistines; antioxidants; chelating agents; pharmacological agents such as vitamin C derivatives; humectants such as glycol; water; water soluble high polymers; polyhydric alcohols; refreshers; and perfumes.

Of the powders that can be added to the cosmetic preparation of the present invention, those normally added to cosmetic compositions for the purpose of coloring effect, makeup effect, ultra-violet radiation shielding effect, and feel adjustment effect such as inorganic particles, photoluminescent particles, organic particles, pigment particles, and composite particles, with no limitations to particle shape, size, and structure, can be given. These particles may be used individually or in combination of two or more. Specifically, inorganic particles such as titanium oxide, ferric ferrocyanide, ultramarine, red iron oxide, yellow iron oxide, black iron oxide, zinc oxide, aluminium oxide, silica, magnesium oxide, zirconium oxide, magnesium carbonate, calcium carbonate, chromium oxide, chromium hydroxide, carbon black, aluminium silicate, magnesium silicate, magnesium aluminium silicate, mica, synthetic mica, synthetic sericite, sericite, talc, kaolin, silicon carbide, barium sulfate, bentonite, smectite, boron nitride, and the like; photoluminescent particles such as bismuth oxychloride, mica titanium, iron oxide coated mica, iron oxide, titanium oxide, organic pigment-treated mica titanium, aluminum powder, and the like; organic particles such as nylon powder, polymethyl methacrylate, acrylonitrile-methacrylate copolymer powder, vinylidene chloride-methacrylic acid copolymer powder, polyethylene powder, polystyrene powder, organopolysiloxane elastomer powder, polymethylsilsesquioxane powder, polytetrafluoroethylene powder, wool powder, silk powder, cellulose crystal, magnesium stearate, zinc stearate, N-acyl-lysine, and the like; pigment particles such as organic tar type pigment, lake pigment, and the like; and composite particles such as mica titanium coated with titanium oxide powder, zinc oxide powder, barium sulfate, titanium oxide containing silica dioxide, zinc oxide containing silica dioxide, and the like can be given. These particles may be used individually or in combination of two or more.

Composite powders made from two or more of the above particles may be used. In addition, particles with the surface treated by a fluorine compound, silicon containing oil agent, metallic soap, wax, surfactant, fat, oil, hydrocarbon, or the like by a conventional method may also be used. The amount of these particles incorporated in the cosmetic preparation of the present invention is preferably 1-90%, although the specific amount varies depending on the purpose of adding the particles and the type of cosmetic preparation.

Oil agents added to conventional cosmetic preparations with the purpose of improving adhesion to the skin, providing emollience, and improving makeup durability may be used in the cosmetic preparation of the present invention. Such oil agents include hydrocarbons, fats and oils, waxes, hardened oils, ester oils, fatty acids, higher alcohols, silicone oils, fluorine-containing oils, and lanolin derivatives, regardless of the origin (animal oils, vegetable oils, or synthetic oils) and the state (solid, half-solid, liquid, or volatile). Specific examples of such oil agents include hydrocarbons such as paraffin wax, ceresin wax, ozokerite, microcrystalline wax, Japanese tallow, montan wax, fisher tropsch wax, polyethylene wax, liquid paraffin, petroleum jelly, squalane, and the like; natural products such as carnauba wax, beeswax, lanolin wax, candelila, and the like; esters such as glyceryl tribehenate, pentaerythritol colophonate, pentaerythritol rhodinate, isopropyl myristate, dialkyl carbonate, glyceryl trioctanoate, diglyceryl tri-isostearate, and the like; alkyl modified silicones such as stearyl siloxane and the like; fatty acids such as stearic acid, 12-hydroxy stearic acid, behenic acid, oleic acid, and the like; higher alcohols such as cetanol, stearyl alcohol, behenyl alchohol, and the like; fats and oils such as olive oil, castor oil, jojoba oil, mink oil, and the like; lanolin derivatives such as isopropyl lanolin fatty acid, lanolin alcohol, and the like; silicone oils such as dimethyl polysiloxane, methylphenyl polysiloxane, and the like; cyclic silicones such as decamethyl cyclopentasiloxane, octamethyl cyclotetrasiloxane, and the like; polyoxyalkylene modified and alkyl modified silicone oils; fluorine-containing oils such as perfluorodecane, perfluorooctane, and the like. These oil agents may be used individually or in combination of two or more.

The amount of these oil agents used in the cosmetic preparation of the present invention is preferably 0.1-50%, although the specific amount varies depending on the purpose of addition and the type of cosmetic preparation.

The cosmetic preparation incorporating the water-containing powder composition of the present invention includes, but is not limited to, makeup such as eye color, foundation, rouge, face powder, skin care cosmetic preparations such as body powder, anti-perspirant powder, whitening powder, milky lotion, cream, beauty lotion, and sunscreen lotion.

The cosmetic preparation of the present invention explained above containing a water-containing powder composition which breaks and releases water when applied with the fingers, palm of the hand, sponge, or chip applicator possesses the characteristics of refreshing feeling and film forming during use.

The water-containing powder composition of the present invention has excellent production stability and storage stability, breaks and releases water when applied with the fingers, palm of the hand, sponge, or chip applicator. The cosmetic preparation including the water-containing powder composition of the present invention, though having a powdery and solid appearance, easily breaks to release water thereby having refreshing feeling and film forming characteristics that cannot be obtained in conventional cosmetic preparations. Also, in addition to cosmetics, the water-containing powder composition of the present invention can be used in a broad range of products including foods, perfumes, agricultural chemicals, and medicines.

### EXAMPLES

The present invention will be described in more detail by way of Examples which should not be construed as limiting the present invention.

### Example 1

### Water-containing powder composition:

Water-containing powder compositions were prepared in the following manner from the components shown in Table 1. The water-containing powder compositions thus prepared were evaluated for "production stability", "storage stability", and "ease of collapsing and releasing water under finger pressure during use" in the following manner. The results are shown in Table 2.

### (Composition)

**Table 1**

| Table 1 | | | | | | | | (weight%) |
|---|---|---|---|---|---|---|---|---|
| | Present Invention | | | | | | | Comparative Example |
| | 1 | 2 | 3 | 4 | 5 | 6 | 7 | 1 |
| 1. Purified water | Balance | Balance | Balance | Balance | Balance | Balance | Balance | Balance |
| 2. Agar | 0.2 | 2 | 2 | - | - | - | 1 | - |
| 3. Gelatin | - | - | - | 3 | - | - | - | - |
| 4. Carageenan | - | - | - | - | 0.05 | 0.05 | - | - |
| 5. Starch | - | - | - | 0.2 | - | - | - | - |
| 6. Carboxy vinyl polymer | - | - | - | - | 0.7 | - | - | - |
| 7. Magnesium sodium silicate *1 | - | - | - | - | - | - | 10 | - |
| 8. Alkyl modified carboxy vinyl polymer *2 | - | - | - | - | | 0.5 | - | - |
| 9. Glycerol | 2 | 2 | 2 | 2 | 2 | 2 | 2 | 2 |
| 10. 1,3-Butylene glycol | 10 | 10 | 10 | 10 | 10 | 10 | 10 | 10 |
| 11. Antiseptic agent | Appropriate amount | Appropriate amount | Appropriate amount | Appropriate amount | Appropriate amount | Appropriate amount | Appropriate amount | Appropriate amount |
| 12. Triethanol amine | - | - | - | - | 0.7 | 0.5 | - | - |
| 13. Liquid paraffin | - | - | - | - | - | 5 | - | - |
| 14. Talc | - | - | - | - | 3 | - | - | - |
| 15. Soybean phospholipid | - | - | - | - | 0.3 | - | - | - |
| 16. Perfume | Appropriate amount | Appropriate amount | Appropriate amount | Appropriate amount | Appropriate amount | Appropriate amount | Appropriate amount | Appropriate amount |
| 17. Hydrophobicized silicic acid anhydride *3 | 3 | 3 | 25 | - | - | - | - | - |
| 18. Hydrophobicized silicic acid anhydride *4 | - | - | - | 3 | 3 | 3 | 3 | 3 |

| | | | | | | | | |
|---|---|---|---|---|---|---|---|---|
| *1: Laponite XLG (manufactured by Laponite Co., Ltd.) *2: Carbopol 1342 (manufactured by Goodrich Chemical Co., Ltd.) *3: AEROSIL R972 (dimethyl dichlorosilane treated silicic acid anhydride manufactured by Nippon Aerosil Co., Ltd.) *4: AEROSIL RX300 (hexamethyl disilazane treated silicic acid anhydride manufactured by Nippon Aerosil Co., Ltd.) | | | | | | | | |

### (Method of preparation)

A. The components 1-12 are mixed and dissolved (or swelled).
B. The components 13-16 are added to A and are mixed by dispersion.
C. B is cooled to -80°C using liquefied nitrogen and freeze-shattered to obtain an aqueous gel powder with a particle diameter of 80 µm.
D. C is added to and mixed with the component 17 or 18 in a stirring vessel while stirring to obtain a water-containing powder composition.

### (Method of evaluation)

### Production Stability:

The water-containing powder composition was prepared five times under the same conditions and the fluctuation of the particle diameter was evaluated according to the following standard.

### Evaluation Standard

⊚ Completely uniform with no fluctuation
○ Nearly uniform with slight fluctuation
Δ Fluctuation is present
× Fluctuation is significant

### Storage stability:

The water-containing powder composition was stored at 40°C for six months and then evaluated according to the following standards.

### Evaluation Standard

⊚ No change in outward appearance after storage
○ Slight association of the particles is noticeable
Δ Association of the particles is noticeable
× Water separation is noticeable

### Ease of collapsing and releasing water under finger pressure during use:

Each water-containing powder composition was pressed with the fingers, and the condition of water release was evaluated according to the following standards.

### Evaluation Standard

⊚ The composition immediately collapses and releases water
○ Although slightly difficult to collapse, the composition releases water
Δ Although difficult to collapse, the composition releases water
× The composition collapses and releases water with difficulty

### (Results)

**Table 2**

| | Production stability | Storage stability | Ease of collapsing and releasing water during use |
|---|---|---|---|
| Example 1 | ○ | ○ | ○ |
| Example 2 | ⊚ | ⊚ | ⊚ |
| Example 3 | ○ | ⊚ | ○ |
| Example 4 | ⊚ | ⊚ | ⊚ |
| Example 5 | ○ | ○ | ○ |
| Example 6 | ○ | ○ | ○ |
| Example 7 | ○ | ○ | ○ |
| Comparative Example 1 | Δ | × | ○ |

As is clear from Table 2, the water-containing powder compositions 1-7 of the present invention have excellent "production stability", "storage stability", and "ease of collapsing and releasing water under finger pressure during use" in comparison to the Comparative Example.

### Example 2

### Water-containing powder composition:

Water-containing powder compositions were prepared from the following components using the following method. The water-containing powder compositions prepared were evaluated as ⊚ for "production stability", "storage stability", and "ease of collapsing and releasing water under finger pressure during use" under the same conditions in Example 1.

| (Component) | weight% |
|---|---|
| 1. Purified water | balance |
| 2. Agar | 1 |
| 3. Magnesium sodium silicate *¹ | 5 |
| 4. Glycerol | 2 |
| 5. 1,3-butylene glycol | 10 |
| 6. Antiseptic agent | appropriate amount |
| 7. Triethanol amine | 1 |
| 8. Stearic acid | 2 |
| 9. Glycol monostearic acid | 1 |
| 10. Cetanol | 1 |
| 11. Dimethyl polysiloxane | 1 |
| 12. Liquid paraffin | 5 |
| 13. 2-ethylhexyl paramethoxy | 1 |
| cinnamic acid | |
| 14. Sorbitan sesquioleate | 0.5 |
| 15. Polyoxyethylene sorbitan monooleate | 0.5 |
| 16. Nylon powder | 1 |
| 17. Perfume | appropriate amount |
| 18. hydrophobic treated silicic acid anhydride *⁴ | 3 |

### (Method of preparation)

A. The components 1-7 are mixed (or swelled) in a solution.
B. The components 8-15 are heated, dissolved, added to A, and emulsified.
C. The components 16-17 are added and mixed by dispersion.
D. C is cooled to -120°C using liquefied nitrogen, and freeze-shattered to obtain an aqueous gel powder with a particle diameter of 80 µm.
E. D is added to and mixed with the component 18 in a stirring vessel while stirring to obtain a water-containing powder composition.

### Example 3

### Deodorant powder:

| (Component) | weight% |
|---|---|
| 1. Aluminum hydroxychloride | 20 |
| 2. Purified water | balance |
| 3. Agar | 3 |
| 4. Propylene glycol | 5 |
| 5. Polyoxyethylene (20 mol) oleyl ether | 0.5 |
| 6. Ethyl alcohol | 5 |
| 7. Hemlock benzal chloride | 0.2 |
| 8. Perfume | 0.3 |
| 9. Hydrophobic treated silicic acid anhydride *⁴ | 5 |

### (Method of preparation)

A. 1-4 are mixed and dissolved.
B. 5-8 are mixed and dissolved.
C. A is added to B and solubilized.
D. C is powderized using a Henschel mixer (manufactured by Mitsui Miike Chemical Industry Co., Ltd.).
E. D is added to and mixed with 9 to obtain a deodorant powder.

### Example 4

### Wine powder:

| (Component) | weight% |
|---|---|
| 1. Purified water | 10 |
| 2. Agar | 1 |
| 3. Wine | 36 |
| 4. Silicic acid anhydride treated with magnesium stearate | 3 |

### (Method of preparation)

A. 1-2 are heated and dissolved, 3 is added and mixed.
B. A is cooled to -80 to -100°C and then freeze-shattered.
C. B is added to and mixed with 4 to obtain a wine powder.

### Preparation Example 1

### Water-containing powder composition (foundation):

| (Component) | weight% |
|---|---|
| 1. Titanium oxide treated with fluorine compound *⁵ | 5 |
| 2. Bengala treated with fluorine compound *⁵ | 0.1 |
| 3. Yellow iron oxide fluorine compound *⁵ | 1 |
| 4. Black iron oxide fluorine compound *⁵ | 0.05 |
| 5. Sericite treated with silicon *⁶ | balance |
| 6. Talc treated with silicon *⁶ | 10 |
| 7. Water-containing powder | 70 |
| composition of Example 1 | |

| | |
|---|---|
| *⁵: powder treated with 5% perfluoroalkyl phosphate *⁶: powder treated with 3% methyl hydrodiene polysiloxane | |

### Preparation Example 2

### Water-containing powder composition (eye shadow)

| (Component) | weight% |
|---|---|
| 1. Water-containing powder composition of Example 1 | balance |
| 2. Partially cross-linked organopolysiloxane swelling material *⁸ | 2 |
| 3. Dimethyl polysiloxane | 1 |
| 4. Silicon treated silicic acid anhydride *⁶ | 5 |
| 5. Silicone treated red pigment no. 202 *⁶ | 0.3 |
| 6. Yellow iron oxide | 1 |
| 7. Titanium mica treated with fluorine compound *⁵ | 5 |
| 8. Talc treated with metallic soap *⁹ | 10 |
| 9. Titanium oxide | 1 |
| 10. Antiseptic agent | proper amount |

| | |
|---|---|
| *⁸ : silicon KSG-18 (manufactured by Shin-Etsu Chemical Co., Ltd.) *⁹ : powder treated with 3% zinc laurate | |

### Preparation Example 3

### Water-containing powder composition (beauty lotion)

| (Component) | weight% |
|---|---|
| 1. Water-containing powder composition of Example 2 | balance |
| 2. Cane sugar fatty acid ester | 1 |
| 3. Dipropylene glycol | 1 |
| 4. Polystyrene powder | 3 |
| 5. Antiseptic agent | appropriate amount |

### Preparation Example 4

### Water-containing powder composition (whitening powder):

| (Component) | weight% |
|---|---|
| 1. Water-containing powder composition of Example 1 | balance |
| 2. Ascorbic acid magnesium phosphate | 1 |
| 3. Ascorbic acid sodium phosphate | 1 |
| 4. Diglycerine | 0.5 |
| 5. Hydrogenated soybean phospholipid | 1 |
| 6. Squalane | 2 |
| 7. Dextrin.fatty acid ester | 0.3 |
| 8. Nylon powder treated with fluorine compound *⁵ | 3 |
| 9. Perfume | appropriate amount |
| 10 Antiseptic agent | appropriate amount |

| | |
|---|---|
| *⁵: powder treated with 5% perfluoroalkyl phosphate | |

### Preparation Example 5

### Water-containing powder composition (sunscreen)

| (Component) | weight% |
|---|---|
| 1. Water-containing powder composition of Example 2 | balance |
| 2. Titanium oxide particles treated with fluorine compound *⁵ | 2 |
| 3. Silicon treated zinc oxide particles *⁶ | 1 |
| 4. 2-Triglyceryl ethyl hexanoic acid | 1 |
| 5. 4-Tert-butyl-4'-methoxy dibenzoylmethane | 0.05 |
| 6. Alkyl polyacrylate powder | 5 |
| 7. Perfume | appropriate amount |
| 8. Antiseptic agent | appropriate amount |

| | |
|---|---|
| *⁵: powder treated with 5% perfluoroalkyl phosphate *⁶: powder treated with 3% methyl hydrodiene polysyloxane | |

### Preparation Example 6

### Water-containing powder composition (body powder)

| (Component) | weight% |
|---|---|
| 1. Water-containing powder composition of Example 3 | balance |
| 2. 1-Menthol | 0.05 |
| 3. Camphor | 0.05 |
| 4. Ethanol | 2 |
| 5. Silicic anhydride | 10 |
| 6. Antiseptic agent | appropriate amount |

## Claims

1. A water-containing powder composition comprising aqueous gel cores coated with hydrophobic particles, wherein the aqueous gel cores are obtainable by
(i) gelling an aqueous phase ingredient with a water-soluble gellant, wherein the aqueous gellantis one or more components selected from the group consisting of agar, gelatin, carageenan, gellan gum, and magnesium sodium silicate, and
(ii) freeze-shattering the gel.

2. The water-containing powder composition of claim 1, wherein the hydrophobic particles have a particle diameter of 1/10 or less of the particle diameter of the aqueous gel cores.

3. The water-containing powder composition of claim 1 or 2, wherein the cores obtainable from the aqueous gel are powdered gel cores.

4. A process for manufacturing a water-containing powder composition, comprising gelling an aqueous phase ingredient with a water-soluble gellant which is one or more component selected from the group consisting of agar, gelatin, carageenan, gellan gum, and magnesium sodium silicate to form aqueous gel cores, and coating the aqueous gel cores with hydrophobic particles.

5. The process for manufacturing the water-containing powder composition of claim 4, wherein the aqueous phase ingredient gelled using a water-soluble gellant is formed into powdered aqueous gel cores by freeze-shattering.

6. A cosmetic preparation comprising the water-containing powder composition according to any one of claims 1 to 3.

7. A method of applying makeup, comprising applying the cosmetic preparation of claim 6 to the skin and applying pressure to cause water to release from said composition.

## Patentansprüche

1. Wasser-enthaltende Pulverzusammensetzung, umfassend wässrige Gelkerne, die mit hydrophoben Teilchen beschichtet sind, wobei die wässrigen Gelkerne erhältlich sind durch
(i) Gelierung einer wässrigen Phase-Komponente mit einem wasserlöslichem Geliermittel, wobei das wässrige Geliermittel eine Komponente ist, die ausgewählt wird aus der Gruppe, bestehend aus Agar, Gelatine, Carageenan, Gellangummi und Magnesiumnatriumslilikat, und
(ii) Gefrierzertrümmern des Gels.

2. Die wasser-enthaltende Pulverzusammensetzung nach Anspruch 1, wobei die hydrophoben Teilchen einen Teilchendurchmesser von 1/10 oder weniger des Teilchendurchmessers der wässrigen Gelkerne aufweist.

3. Die wasser-enthaltende Pulverzusammensetzung nach Anspruch 1 oder 2, wobei die Kerne, die aus einem wässrigen Gel erhältlich sind, pulverisierte Gelkerne sind.

4. Verfahren zur Herstellung einer wasser-enthaltenden Pulverzusammensetzung, umfassend eine Gelierung eines wässrigen Phasebestandteils mit einem wasserlöslichem Geliermittel, das eine Komponente ist, die ausgewählt wird aus der Gruppe, bestehend aus Agar, Gelatine, Carageenan, Gellangummi und Magnesiumnatriumsilikat, um wässrige Gelkerne zu bilden und Beschichtung der wässrigen Gelkerne mit hydrophoben Teilchen.

5. Das Verfahren zur Herstellung einer wasser-enthaltenden Pulverzusammensetzung nach Anspruch 4, wobei der wässrige Phasebestandteil, der unter Verwendung eines wasserlöslichen Geliermittels Geliert wird, in pulverisierte wässrige Gelkerne umgewandelt wird durch Gefrierzertrümmerung.

6. Kosmetische Zubereitung, umfassend die wasserhaltige Pulverzusammensetzung nach einem der Ansprüche 1 bis 3.

7. Verfahren zur Anwendung von Make-up, umfassend eine Anwendung der kosmetischen Zubereitung nach Anspruch 6 auf die Haut und Anwendung von Druck, um Wasser aus der Zusammensetzung austreten zu lassen.

## Revendications

1. Composition de poudre contenant de l'eau, comprenant des coeurs de gel aqueux revêtus de particules hydrophobes, dans laquelle les coeurs de gel aqueux peuvent être obtenus par
(i) gélification d'un ingrédient de phase aqueuse avec un gélifiant soluble dans l'eau, le gélifiant aqueux étant un ou plusieurs composants choisis dans l'ensemble constitué par la gélose, la gélatine, la carraghénane, la gomme gellane, et le sodosilicate de magnésium, et
(ii) cryoclastie du gel.

2. Composition de poudre contenant de l'eau selon la revendication 1, dans laquelle les particules hydrophobes ont une granulométrie représentant 1/10 ou moins de la granulométrie des coeurs de gel aqueux.

3. Composition de poudre contenant de l'eau selon la revendication 1 ou 2, dans laquelle les coeurs pouvant être obtenus à partir du gel aqueux sont des coeurs de gel en poudre.

4. Procédé pour fabriquer une composition de poudre contenant de l'eau, comprenant la gélification d'un ingrédient de phase aqueuse avec un gélifiant soluble dans l'eau, qui est un ou plusieurs composants choisis dans l'ensemble constitué par la gélose, la gélatine, la carraghénane, la gomme gellane, et le sodosilicate de magnésium, pour former des coeurs de gel aqueux, et le revêtement des coeurs de gel aqueux avec des particules hydrophobes.

5. Procédé pour fabriquer une composition de poudre contenant de l'eau selon la revendication 4, dans lequel l'ingrédient de phase aqueuse gélifié par utilisation d'un gélifiant soluble dans l'eau est mis sous la forme de coeurs de gel aqueux en poudre par cryoclastie.

6. Préparation cosmétique comprenant la composition de poudre contenant de l'eau selon l'une quelconque des revendications 1 à 3.

7. Procédé pour appliquer un maquillage, comprenant l'application de la préparation cosmétique de la revendication 6 à la peau et l'application d'une pression pour provoquer la libération d'eau à partir de ladite composition.
